# EUROPEAN PATENT SPECIFICATION

(11) **EP 4 154 876 B1**
(45) Date of publication and mention of the grant of the patent: **10.07.2024**
(21) Application number: 21198873.8
(22) Date of filing: 24.09.2021
(51) Int. Cl.: A61K 31/166, A61K 31/495, A61K 45/06, A61P 9/00, A61P 9/14, A61K 31/7088, A61K 31/7105

(54) **CBX7 INHIBITOR FOR USE IN THE TREATMENT AND/OR PREVENTION OF A BLOOD VESSEL DISORDER**
CBX7-INHIBITOR ZUR VERWENDUNG BEI DER BEHANDLUNG UND/ODER VORBEUGUNG EINER BLUTGEFÄSSSTÖRUNG
INHIBITEUR DE CBX7 À UTILISER DANS LE TRAITEMENT ET/OU LA PRÉVENTION D'UNE MALADIE DES VAISSEAUX SANGUINS

(43) Date of publication of application: 29.03.2023
(73) Proprietor: Universität Potsdam, 14469 Potsdam (DE)
(72) Inventor: Seyfried, Salim, Potsdam (DE)
(74) Representative: Hertin und Partner Rechts- und Patentanwälte PartG mbB

(56) References cited:
- WO-A1-2019/115472
- REN CHUNYAN ET AL: "Structure-Guided Discovery of Selective Antagonists for the Chromodomain of Polycomb Repressive Protein CBX7", ACS MEDICINAL CHEMISTRY LETTERS, vol. 7, no. 6, 9 June 2016 (2016-06-09), US, pages 601 - 605, XP055902552, ISSN: 1948-5875, Retrieved from the Internet <URL:https://pubs.acs.org/doi/pdf/10.1021/acsmedchemlett.6b00042> DOI: 10.1021/acsmedchemlett.6b00042

## Description

The present invention is in the field of treating a blood vessel disorder, preferably vascular malformation.

The invention relates to a chromobox protein homolog 7 (CBX7) inhibitor for use in the treatment and/or prevention of a blood vessel disorder, preferably vascular malformation. The invention further relates to a CBX7 inhibitor for treating and/or preventing a blood vessel disorder in combination with a pharmaceutically acceptable carrier.

### BACKGROUND OF THE INVENTION

A blood vessel disorder is an abnormal condition of the blood vessels. The body uses blood vessels to circulate blood through itself. Problems along this vast network can cause severe disability and death. Blood vessel disorder is mostly referred to vascular malformation.

Cerebral cavernous malformations (CCMs) are thought to be monogenic diseases of brain capillaries that can lead to stroke. A recent study by Ren et al (Nature 2021, Jun;594(7862):271-276. doi: 10.1038/s41586-021-03562-8. Epub 2021 Apr 28. PMID: 33910229 ) identified somatic gain-of-function mutations in *PIK3CA* and loss-of-function mutations in the CCM complex in the same cells in a majority of human CCMs. Cerebral cavernous malformation is one of the most common vascular malformations with a prevalence of 0.4 to 0.5 percent of the population. The familiar form of the disease is caused by a "two-hit" mechanism in which heterozygous carriers with a heterozygous germline mutation in one of the genes CCM1/KRIT1 (Duboysky et al., 1995), CCM2/MGC4607, or CCM3/PDCD10 (Craig et al., 1998) acquire another somatic mutation in the other allele (Akers et al., 2009). CCM defects occur primarily within the cerebral capillary vasculature and are characterized by a mulberry-like appearance. Clinical symptoms range from neuronal defects such as seizures and headaches to hemorrhagic stroke (Spiegler et al., 2018).

Currently, increasing interest is directed toward potential pharmacologic approaches for the treatment of CCM. Some drug screens have been performed in the past including by the research group of Seyfried (Otten et al., 2018). It has been revealed that a variety of molecular factors play a potential role in the development of CCM disease.

Among others, pathological alterations in biomechanical signal transduction pathways have also been linked to etiologies of various vascular diseases (Baeyens et al., 2016b), including cerebral cavernous malformations (CCM) (Macek Jilkova et al., 2014; Mleynek et al., 2014; Renz et al., 2015). The study with various model organisms has also demonstrated a critical role for the mitogen-activated protein kinase (MAPK) signaling cascade pathway involving Mekk3 and Erk5 (Uhlik et al., 2003; Zhou et al., 2015, 2016; Fisher et al., 2015; Cuttano et al., 2016). These activate a signaling cascade via the transcription factors Klf2/4, which play an important function in the response to biomechanical stimuli in endothelial cells (Uhlik et al., 2003; Maddaluno et al., 2013; Renz et al., 2015; Zhou et al. 2015; Zhou et al., 2016; Cuttano et al., 2016; Chapman et al. 2019; Castro et al., 2019).

Pharmacological inhibition of MAPK signaling and endothelial-specific knockouts of Mekk3 (Zhou et al., 2016) and Klf2/4 suppressed vascular lesion formation in CCM mouse models (Zhou et al., 2015, 2016; Cuttano et al., 2016). Interestingly, Klf2 also plays a role in endothelial-mesenchymal transition (endMT), which contributes to CCM lesions (Maddaluno et al., 2013). During endMT, endothelial cells break cell-cell junctions, resulting in loss of endothelial integrity, and acquire mesenchymal properties. This can cause affected blood vessels to hemorrhage. In this context, the first compounds are now being tested in preclinical and clinical applications (Abdelilah-Seyfried et al., 2020).

Although a pharmacologic approach for the treatment of CCM has become a field of increasing interest and gained some initial success, surgical resection is still the common treatment for CCM. Surgery can be performed for symptomatic cavernomas, but this is not always possible due to the location and accessibility of the lesion. Other therapeutic measures are currently not available.

Accordingly, there remains a need in the art for the development of effective pharmacological approaches for treating and/or preventing blood vessel disorder, such as in particular cerebral cavernous malformations.

### SUMMARY OF THE INVENTION

In light of the prior art the technical problem underlying the present invention is to provide means for treating and/or preventing a blood vessel disorder, preferably a vascular malformation.

This problem is solved by the features of the independent claim. Preferred embodiments of the present invention are provided by dependent claims.

The invention therefore relates to a chromobox protein homolog 7 (CBX7) inhibitor for use in the treatment and/or prevention of a blood vessel disorder.

CBX7 is a component of Polycomb repressive complex (PRC1). The PRC1 complex acts as a repressor of transcription factors and other genes (Simon and Kingston, 2009). This occurs through direct binding of Cbx7 to H3K27me3 histone modifications and recruitment of the PRC1 complex (Zhen et al., 2019) and concomitant alteration of the chromatin scaffold. These changes in the chromatin scaffold result in a repressive effect on transcriptional processes. A role for CBX7 has been particularly discussed in tumor biology, where Cbx7 has been implicated in epithelial-mesenchymal transition (Bao et al, 2017; Federico et al, 2019; Zhao et al, 2020) and has been described as a tumor repressor (Forzati et al, 2012). Moreover, PRC1 has been described as an important therapeutic target in the context of metastasis in prostate cancer (Su et al., 2019). Overexpression of CBX7 has been linked to an overproliferation of hematopoietic stem cells in acute myeloid leukemia (Jung et al. 2019).

The present invention is based on the surprising finding that there is a link between the epigenetic regulator protein CBX7, which acts as a member of the complex PRC1, and the pathogenesis of blood vessel disorders. This link has been found through an analysis of the whole transcriptome profile of zebrafish *ccm2* mutants, which represent a well-established animal model of CCM. The mRNA and protein levels of CBX7 are strongly activated in the zebrafish *ccm2* mutant model. Even more surprisingly, it was found that knockout of CBX7 as well as pharmacological inhibition of CBX7 suppress the formation of CCM phenotypes in zebrafish models of CCM. These findings imply an important role of the epigenetic regulator protein CBX7 and the PRC1 complex in CCM pathology and identified CBX7 inhibition as a suitable treatment/prevention of CCM related blood vessel disorders. To the knowledge of the inventors, there has neither been a suggestion in the art regarding a link between disease CCM and the epigenetic regulator protein Cbx7. Also, there is no hint in the art that there may be a functional link between CCM and Polycomb Repressive Complex 1 (PRC1), in which the protein Cbx7 acts. It is advantageous that the treatment of blood vessel disorder can be non-invasive compared to surgical resection. A non-invasive treatment can largely reduce pain and discomfort during treatment in comparison to surgery and is further associated with a comparably low risk of complications.

A further advantage of the invention, which is based on the described surprising findings, is that symptomatic cavernomas, which are located in some inaccessible positions for a surgical resection, can be treated with the method according to the invention.

The prophylactic treatment of CBX7 may be used effectively to prevent or slow down the progression of established blood vessel disorder, in particular vascular lesions, in a subject or it may be used to prevent the development of vascular lesions in a subject at risk of developing a blood vessel disorder.

In embodiments, the CBX7 inhibitor is for use in the treatment treatment and/or prevention of a blood vessel disorder in a subject, wherein preferably the subject is a mammal, most preferably a human subject.

In embodiments, the inhibitor of CBX7 as used herein is for treating a blood vessel disorder associated with an enlarged heart, stroke, hyperthermia, palpable pulsatility, phleboedema, ulcers, gangrenous changes, hemorrhage, chronic venous insufficiency, arterial ischemia, tachycardia, tachyarrhythmia, cardiomegaly, dilatation or hypertrophy, high-output cardiac failure. In a preferred embodiment, the blood vessel disorder is a vascular malformation.

As used herein, vascular malformation refers to blood vessel or lymph vessel abnormality. In embodiments, the vascular malformation is characterized by or associated with endothelial-mesenchymal transition. In embodiments, the vascular malformation is within the central nervous system and/or the retina vasculature. In preferred embodiment, the vascular malformation is within the brain.

In embodiments, the vascular malformation is cerebral cavernous malformation (CCM), arteriovenous malformation (AVM), capillary malformation (CM), lymphedema (LE), or hereditary hemorrhagic telangiectasia (HHT), preferably CCM. In embodiments, the vascular malformation is arteriovenous malformation (AVM) or hereditary hemorrhagic telangiectasia (HHT).

In a preferred embodiment, the vascular malformation is cerebral cavernous malformation (CCM) which is the most prevalent type of vascular malformation within the central nervous system. In CCM, the absence of pericytes and the deficiency of the tight and adherens junctions results in a single layer of endothelial cells (as observed by histology), which leads to the impairment of the blood-brain barrier (BBB). CCM occurs in two forms, sporadic and familial, wherein the latter has an autosomal dominant mode of inheritance with incomplete penetrance and viable clinical expressivity. There are three genes considered to be associated with CCM, namely, *CCM1*/*KRIT1* coding for CCM1 protein, *CCM2*/*MGC4607* coding for CCM2 protein, *CCM3*/*PDCD10* coding for CCM3.

In embodiments, the invention relates to an inhibitor of CBX7 for use in treating a medical condition associated with CCM, such as intracerebral hemorrhage (ICH) and seizures, as well as symptoms or conditions associated with CCM including recurrent headaches, focal neurological deficits, vertigo, and paresis.

In one embodiment, the blood vessel disorder is associated with increased CBX7 mRNA and/or protein expression in tissues of the cardiovascular system, such as arteries, capillaries, veins, portal veins, heart, coronary vessels, lungs, systemic circulation, brain, kidneys and lymphatic system.

The analysis of whole transcriptome of zebrafish *CCM2* mutants, which is an animal model for blood vessel disorders and in particular CCM, revealed the surprising finding of an increase of *cbx7* mRNA and CBX7 protein levels in the tissue of the cardiovascular system that is associated with blood vessel disorder. This surprising finding identifies CBX7 as a therapeutic target for the treatment and/or prevention of blood vessel disorders, in particular vascular malformations such as CCM, wherein CBX7 can be targeted on a transcriptional, translational, post-translational or protein level.

In embodiments, the CBX7 inhibitor is used for treating acute vascular lesions. In such embodiments, the treatment with CBX7 inhibitor is for stopping the bleeding and other acute symptoms relating to (established) vascular lesions. Accordingly, the use of a CBX7 inhibitor for treating acute vascular lesions is a therapeutic treatment. In a preferred embodiment, the administration of the CBX7 inhibitor leads to regression of vascular lesions. In other preferred embodiments, the administration of the CBX7 inhibitor prevents progression of vascular lesion, such as the enlargement of existing vascular lesions or the formation of additional vascular lesions.

In other embodiments, the invention relates to a CBX7 inhibitor for use in the treatment of chronic lesions. In such embodiments, the CBX7 inhibitor is mostly used preventively. For example the inhibitor may be used for treating an asymptomatic patient, who is known or expected to sporadically develop vascular lesions in the (near) future, in particular if no treatment is administered. In a preferred embodiment, the administration of the CBX7 inhibitor prevents formation of vascular lesions associated with a blood vessel disorder.

In one embodiment, the CBX7 inhibitor blocks/prevents the binding of CBX7 to histone modifications on the genomic DNA. In other embodiments, a CBX7 inhibitor interrupts or disrupts the interaction between the chromodomain of CBX7 and H3K27me3 histone on the genomic DNA. In embodiments, the CBX7 inhibitor blocks the binding to other proteins that carry trimethylated residues (non-histone) with which CBX7 would otherwise associate. In one embodiment, the CBX7 inhibitor blocks the proliferation of mutant cells or stem cells in the diseased vasculature.

In embodiments, the conformation of CBX7 protein is changed by the inhibitor and thus the activity of the CBX7 protein is impaired which reduces CBX7 activity, such as the binding of CBX7 to the H3K27me3 histone on the genomic DNA. In other preferred embodiment, the conformation of CBX7 protein is changed by the inhibitor and thus the specificity of the CBX7 protein binding to H3K27me3 histone is reduced or even abolished. This can prevent binding of CBX7 to genomic DNA and abolished its physiological activity.

In embodiments, the CBX7 inhibitor interferes with CBX7 activity. This can occur on any regulatory level. In embodiments, a CBX7 inhibitor prevents transcription of CBX7 mRNA. In embodiments, a CBX7 inhibitor leads to degradation of CBX7 mRNA and/or interferes with CBX7 mRNA translation to CBX7 protein. In embodiments, a CBX interferes with CBX7 protein activity and/or function, or accelerates CBX7 protein degradation.

In embodiments, the CBX7 inhibitor is a CBX7-suppressive oligonucleotide, such as an antisense oligonucleotide, a microRNA (miR), an shRNA, an siRNA, or a guide-RNA in conjunction with an RNA-guided endonuclease (for example by acting through the CRISPR/Cas9 system), or a nucleic acid molecule suitable for expression of a CBX7-suppressive oligonucleotide, such as an expression plasmid or a viral vector encoding a CBX7-suppressive oligonucleotide. In embodiments, a CBX7 inhibitor acts by means of RNA interference technology, such as an siRNA or shRNA.

In embodiments, an inhibitor act by downregulating the levels of CBX7 post-translationally, for example ubiquitination of CBX7. In embodiment, a CBX7 inhibitor induces a knock-out of the *cbx7* gene in the genomic DNA, for example by means of a *cbx7-specific* gRNA in conjunction with an RNA guided endonuclease, for example by using the CRISPR/Cas9 system.

A CBX7 inhibitor as used herein can be selected from, but is not limited to, the group consisting of MS37452, MS351, UNC3866, UNC4976, adapted L3MBTL1 inhibitor, nucleic acid molecules blocking or preventing Cbx7 expression, such as a CBX7-specific shRNA or a CBX7-specific gRNA used in conjunction with an RNA guided endonuclease, or any combination thereof.

In one embodiment, the CBX7 inhibitor can be used in combination with a pharmaceutically acceptable carrier. In one aspect, the invention relates to a pharmaceutical composition comprising a CBX7 inhibitor as described herein for use in the treatment of a blood vessel disorder as described herein.

A pharmaceutical composition of the invention preferably comprises a CBX7 inhibitor in combination with a pharmaceutically acceptable carrier which may be any of those known in the art or devised hereafter and suitable for the intended use. The pharmaceutical composition of the invention may include other ingredients, including dyes, preservatives, buffers and antioxidants. In some embodiment, the pharmaceutical composition as used herein can be formulated for administration by oral, intravenous, intramuscular, or subcutaneous, topical routes. In another embodiment, the pharmaceutical composition or the CBX7 inhibitor can be directly applied to the location of vascular lesion.

In embodiments of the invention, the treatment and/or prevention of a blood vessel disorder further comprises simultaneous or sequential administration of one or more other active agents useful for treating the blood vessel disorder.

The various aspects of the invention are based on and/or are linked by the common and surprising finding that inhibition of CBX7 activity is effective in treating and/or preventing blood vessel disorders, such as vascular malformation including CCM. The various features and advantages of the invention that are disclosed in one aspect of the invention are herewith also disclosed in the context of the other aspects of the invention described herein.

### DETAILED DESCRIPTION OF THE INVENTION

The invention relates to a chromobox protein homolog 7 (CBX7) inhibitor for use in the treatment and/or prevention of a blood vessel disorder, preferably vascular malformation. The invention further relates to a CBX7 inhibitor for treating and/or prevention of a blood vessel disorder in combination with a pharmaceutically acceptable carrier. Accordingly, the invention also relates to a pharmaceutical composition comprising a CBX7 inhibitor and a pharmaceutically acceptable carrier.

CBX7 is a component of Polycomb repressive complex (PRC1). Polycomb group (PcG) proteins function as gene repressors and are involved in the regulation of many processes in stem cell characteristics and differentiation into functional cells. PcG protein are found in several multiprotein complexes, the best characterized of which are Polycomb repressive complexes 1 and 2 (PRC1 and PRC2). PRC1 is required to maintain the transcriptionally repressive state of many genes, including Hox genes, throughout development. PRC1 acts via chromatin remodeling and modification of histones.

Polycomb repressive complexes 1 (PRC1) contains a single representative of the Polycomb chromobox (CBX) family member, either CBX2, CBX4, CBX6, CBX7 or CBX8. The CBX family has important role in the epigenetic regulation in many cell types and are also called epigenetic regulator. CBX family members are involved for example in cancer progression and the Ink4a/ARF/Ink4b locus encoding three tumor suppressors is one of the earliest identified and the most well-known targets for CBX-containing PRC1 complex.

In the context of the invention, the terms Polycomb repressive complex and Polycomb repressor complex are interchangeable.

CBX7 refers to chromobox protein homolog 7 or chromobox 7, UniprotKB-O95931, which is a component of Polycomb repressive complex (PRC1). In the context of the invention, CBX7 refers to all the orthologs, such as CBX7a in zebrafish. CBX7 refers also to a *cbx7* gene encoding the CBX7 protein as well the gene loci in the *cbx7* gene.

CBX7 is a chromobox domain-containing protein that belongs to the polycomb repressive complex 1 (PRC1). Other members of this PRC1 complex include polycomb group RING finger 3/5 (PCGF3/5), ring finger protein 1 (RING1B), B lymphoma Mo-MLV insertion region 1 homolog (BMI1), polycomb group ring finger2 (Mel-18) and casein kinase 2 (CK2). The association of CBX7 with Mel-18 and BMI1 is of particular interest. Knockout studies have shown that Mel-18 is essential for GATA3 transcription and Th2 differentiation, whereas BMI1 is important for GATA binding protein 3 (GATA3) stability and type 2 cytokine expression. BMI1-/- mice are unable to mount allergic/eosinophilic airway inflammation. MEK/ERK phosphorylates CBX7 at T118, which stabilizes its interaction with Mel-18 and BMI1. Thus, CBX7 and MEK2 could jointly promote type 2 cytokine production through recruitment of Mel-18 and BMI1 under the protagonistic milieu of thymic stromal lymphopoietin (TSLP) and dexamethasone (Dex). CBX7 directly upregulates positive regulators of lymphoid cells: Fos, FosB, Egr1, and TRIM24. CBX7 downregulates GR effectors-HDAC2, annexin A1, and protein phosphatase 2A. Overexpression of CBX7 has been linked to an overproliferation of hematopoietic stem cells in acute myeloid leukemia (Jung et al. 2019).

Chromodomain (ChD) of CBX7 is associated with the remodelling and manipulation of chromatin. Methyl-lysine recognition by the chromodomain represents a fundamental mechanism in the epigenetic control of gene transcription in chromatin. One premier example involves histone H3 lysine 27 trimethylation (H3K27me3), an absolutely conserved epigenetic mark that signals gene transcriptional repression in all eukaryotes. Two conserved Polycomb repressive protein complexes (PRCs) carry out H3K27me3-directed gene transcriptional repression, in that PRC2 first establishes H3K27me3 modification at a target gene site by its lysine methyltransferase EZH2,5, 6 and PRC1 follows by binding H3K27me3 via a ChD of its chromobox (CBX) protein, which results in chromatin condensation and target gene repression. Long non-coding RNAs participate directly in gene transcriptional repression by facilitating recruitment of the PRCs to their target gene loci in chromatin.

Inhibition of CBX7 refers to any kind of interference with the activity of CBX7. In one embodiment, inhibition of CBX7 refers to negative modulation, i.e. inhibition, of the function of CBX7 in PRC1. In one embodiment, the function of the chromodomain of CBX7 is negatively modulated. In embodiments, inhibition of CBX7 relates to an increase of non-specific binding to DNA and RNA. In embodiments, inhibition of CBX7 relates to negative modulation, i.e. downregualation, of the protein level and/or mRNA level of CBX7. Inhibition of CBX7 can further refer to displacing CBX7 from its target gene locus, which may result in transcriptional de-repression of target genes. In a preferred embodiment, CBX7 inhibition relates to an interrupted interaction between CBX7/ChD and methyl-lysine which blocks the interaction between PRC1 complex and chromatin or leads to a weak binding of PRC1 complex and chromatin.

As used herein, the term "inhibitor of CBX7" or "CBX7 inhibitor" shall mean substance, compound, molecule or system which is capable of interfering with the activity of CBX7. In one embodiment, a CBX7 inhibitor modulates the role of CBX7 in PRC1 complex negatively. In one embodiment, a CBX7 inhibitor modulates the function of the chromodomain of CBX7 negatively. In another embodiment, a CBX7 inhibitor can displace the CBX7 from its target gene locus and inducing transcriptional de-repression of target genes. In one embodiment, a CBX7 inhibitor disrupts the interaction between CBX7 and H3K27me3 Histones on genomic DNA. In another embodiment, the inhibitor of CBX7 blocks the interaction between CBX7/ChD and methyl-lysine which blocks the interaction between PRC1 complex and chromatin or leads to a weak binding of PRC1 complex and chromatin. In one embodiment, a CBX7 inhibitor negatively regulates the translation of the CBX7 mRNA. In embodiments, a CBX7 inhibitor functions on the post-translational level, for example by accelerating or inducing CBX7 protein degradation. In another embodiment, a CBX7 inhibitor functions at a RNA level and reduces the mRNA levels of CBX7, such as a CBX7-specific siRNA or shRNA. In some embodiments, a CBX7 inhibitor functions at DNA levels which leads to knock-down or know-out of CBX7, such as via CRISPR-Cas9.

In embodiments, a CBX7 inhibitor can be a compound, a modulator or a ligand or a small molecule inhibitor or antagonist or an antibody binding to CBX7 or a CBX7 siRNA, CBX7 shRNA, or any combinations thereof.

In embodiments, a CBX7 inhibitor can be a CBX7-suppressive oligonucleotide, such as an antisense oligonucleotide, a microRNA (miR), an shRNA, an siRNA, or a guide-RNA in conjunction with an RNA-guided endonuclease, or a nucleic acid molecule suitable for expression of a CBX7-suppressive oligonucleotide, such as an expression plasmid or viral vector encoding a CBX7-suppressive oligonucleotide. In embodiments, the CBX7 suppressor can be a system including several components, such as for example a guide RNA in conjunction with an RNA-guided endonuclease, for example Cas9, which may function for example by generating a functional knock-out of the CBX7 gene in the genomic DNA of a cell by means of the CRISPR/Cas9 system. In such a context, a CBX7 inhibitor system can comprise several components that act together to generate a functional inhibition, for example a function gene knock-out, of CBX7. The skilled person in view of the present disclosure and the common general knowledge is aware of suitable system components that may lead to functional inhibition of CBX7 and that can be used as a CBX7 inhibitor in the sense of the invention.

In embodiments, a CBX7 inhibitor can be a compound, such as a biological or chemical molecule, such as a small molecule, a protein, a nucleic acid, chemicals, inorganic molecules, organic molecules, cDNA encoding a protein, a secreted protein, a large molecule, an antibody, a morpholino, a triple helix molecule, a peptide, siRNA, shRNA, miRNA, antisense RNA, ribozyme or any other compound or combination of comounds that can be envisioned to be used as a functional inhibitor of CBX7 in the treatment of a subject.

Certain CBX7 inhibitors have been described by Ren et al (ACS Med Chem Lett. 2016 Jun 9; 7(6): 601-605. Published online 2016 Feb 29. doi: 10.1021/acsmedchemlett.6b00042) and are a known class of inhibitors.

Non-limiting examples of CBX7 inhibitors are MS37452, MS351, adapted L3MBTL1 inhibitor, UNC3866 and UNC4976.

MS37452 binds to CBX7, changes its conformation, and blocks the interaction between PRC1 complex and H3K27me3 Histones. MS37452 is a molecule according to the following formula:

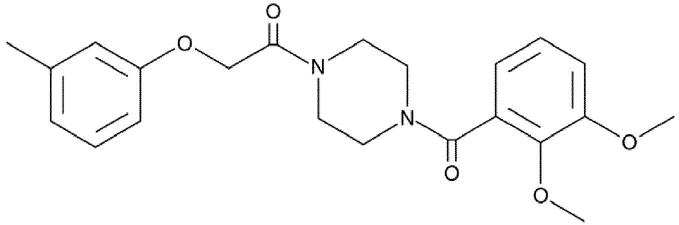

MS351 is a iminobenzimidazole compound binds and forms a ternary complex with CBX7/ChD. MS351 selectively binds to the CBX7ChD alone with modest affinity (estimated *K_{d}* of approximately 500 µM, as determined by NMR titration), but does not bind ChDs from other CBX proteins including CBX1/3/5 (HP1β/γ/α) and CBX2/4/6/8, as described by Ren et al (ACS Med Chem Lett. 2016 Jun 9; 7(6): 601-605. Published online 2016 Feb 29. doi: 10.1021/acsmedchemlett.6b00042). MS351 likely blocks H3K27me3 binding to the CBX7ChD in its functionally active form, i.e., bound to RNA.

Adapted L3MBTL1 inhibitor works as a small-molecule CBX antagonist. Adaptation of L3MBTL1 inhibitors to create small-molecule CBX7 antagonists has been described by Simhadri et al (ChemMedChem. 2019 Aug 6;14(15):1444-1456. doi: 10.1002/cmdc.201900021. Epub 2019 Jul 22.).

UNC3866 is a compound according to the following formula:

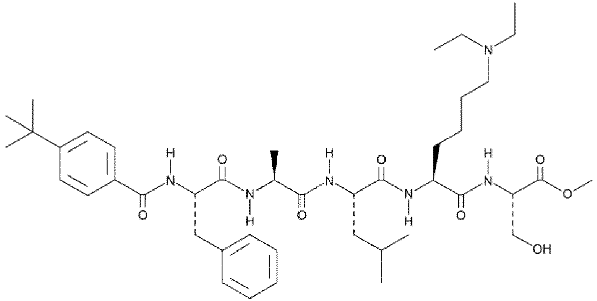

UNC3866 is a potent antagonist of methyl-lysine reading function of the Polycomb CBX and CDY families of chromodomains. UNC3866 binds the chromodomains of CBX4 and CBX7 most potently with a K_{d} of about 100 nM for each and is 6- to 18-fold selective versus seven other CBX and CDY chromodomains while being highly selective versus >250 other protein targets. UNC4976 is an allosteric modulator which antagonizes H3K27me3-specific recruitment of CBX7 to target genes while increasing non-specific binding to DNA and RNA.

UNC4976 is a positive allosteric modulator (PAM) peptidomimetic of CBX7 chromodomain binding to nucleic acids. UNC4976 simultaneously antagonizes H3K27me3-specific recruitment of CBX7 to target genes while increasing non-specific binding to DNA and RNA. It has been described by Lamb et al (Cell Chemical Biology 26, 1365-1379, October 17, 2019, doi.org/10.1016/j.chembiol.2019.07.013) and has the following formula:

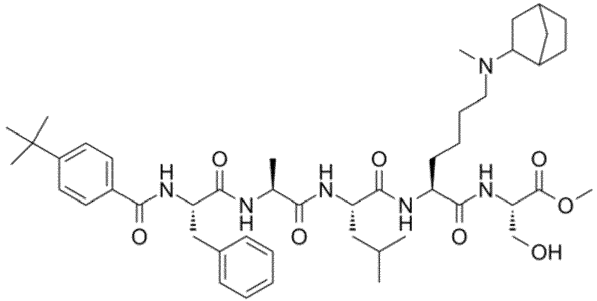

As used herein, the terms "blood vessel disorder", "blood vessel disease", or "vascular disease" are used interchangeably and refer to vascular diseases which is a subgroup of cardiovascular disease. The term "vascular lesion" can relate to vascular diseases and vascular malformation, which is a blood vessel abnormality. In general, the term "vascular lesion" describes any kind of unphysiological vascular formation or phenotype.

Disorders in the vast network of blood vessels can cause a range of health problems which can be severe or prove fatal. There are several types of vascular disease, which is a subgroup of cardiovascular disease, and the signs and symptoms depend on which type, among them are: Erythromelalgia is a rare peripheral vascular disease where syndromes include burning pain, increased temperature, erythema and swelling, of mainly the hands and feet are affected. Peripheral artery disease happens when atheromatous plaques build up in the arteries that supply blood to the arms and legs, plaque causes the arteries to narrow or become blocked. Renal artery stenosis is the narrowing of renal arteries that carry blood to the kidneys from the aorta. Buerger's disease is due to small blood vessels that inflame and swell, vessels then narrow or are blocked by blood clots. Raynaud's disease is a rare peripheral vascular disorder of constriction of the peripheral blood vessels, in the fingers and toes when the person is cold. Disseminated intravascular coagulation is a widespread activation of clotting in the smaller blood vessels. Cerebrovascular disease is a group of vascular diseases that affect brain function.

As used herein the term "cardiovascular system" refers to an organ system that permits blood to circulate and transport nutrients, oxygen, carbon dioxide, hormones, and blood cells to and from the cells in the body to provide nourishment and help in fighting diseases, stabilized temperature, and pH, and maintain homeostasis. The cardiovascular system includes arteries, capillaries, veins, portal veins, heart, coronary vessels, lungs, systemic circulation, brain, kidneys and lymphatic system. The terms circulatory system or vascular system may be used in the same sense.

In the context of the invention, increased cbx7 mRNA and/or CBX7 protein expression in the tissue of the cardiovascular system has been observed.

Tissues of the cardiovascular system include the lymphatic system, which circulates lymph. The passage of lymph for example takes much longer than that of blood. Blood is a fluid consisting of plasma, red blood cells, white blood cells, and platelets that is circulated by the heart through the vertebrate vascular system, carrying oxygen and nutrients to and waste materials away from all body tissues. Lymph is essentially recycled excess blood plasma after it has been filtered from the interstitial fluid (between cells) and returned to the lymphatic system. The cardiovascular system comprises the blood, heart, and blood vessels. The lymph, lymph nodes, and lymph vessels form the lymphatic system, which returns filtered blood plasma from the interstitial fluid (between cells) as lymph.

In the context of the invention, vascular diseases comprise also vascular diseases with a clonal nature of the disease such as in CCM, HHT, Tie2, RASA1, or Marfan syndrome.

Vascular malformation is a vascular disease and refers to conditions in which blood vessel or lymph vessel abnormality occur. It can be characterized by or associated with endothelial-mesenchymal transition. In embodiments, In the vascular malformation, endothelial-mesenchymal transition is present. In other preferred embodiments, the vascular malformation is within the central nervous system and/or the retina vasculature. In a more preferred embodiment, the vascular malformation is within the brain. In some embodiments, the pathology of vascular malformation is further selected from the group consisting of fibrodysplasia, ossificans progressive, cardiac fibrosis, kidney fibrosis, pulmonary fibrosis and cerebral cavernous malformation.

Vascular lesions include CCM lesions that consist of grossly dilated, capillary-like structures lined by endothelium and lacking mature vascular wall structure. Lesions can be found in ~0.5% of the general population and affected individuals have a lifetime risk of focal neurological deficits and hemorrhagic stroke. Sporadic lesions are typically solitary, whereas familial CCM is characterized by multifocal cerebral lesions in the setting of mutations in one of three genes encoding KRIT1 (CCM1), CCM2 and PDCD10 (CCM3) or other genes such as the gene encoding Heg1.

Cerebral cavernous malformations (CCMs) are a common vascular malformation with a prevalence of 0.1%-0.5% in the human population. CCMs arise primarily in the brain as thin-walled, dilated blood vessels that cause seizures, headaches and stroke in midlife, often in association with focal hemorrhage. Familial CCM shows an autosomal dominant pattern of inheritance and is caused by loss-of-function mutations in three genes: KRIT1 (also known as CCM1), CCM2 (also known as malcalvernin and osm) and PDCD10 (also known as CCM3). The CCM proteins are putative adaptor proteins that interact biochemically leading to the formation of a CCM complex and participate in a signaling pathway that is not yet fully characterized. A clue to the role of CCM protein signaling in the cardiovascular system comes from genetic studies in zebrafish, which reveal that loss of *krit1, ccm2* or *heg1* (encoding the type I transmembrane receptor heart of glass) results in a dilated heart phenotype early in development. This phenotype is characterized by heart failure associated with enlarged cardiac chambers in which the endocardium is covered by a thin layer of myocardial cells. Heg1 has been shown to also interact with the CCM complex comprising CCM1, CCM2 and/or CCM3 and functional Heg1 is required for a functional CCM complex. Expression of heg1, krit1 and ccm2 mRNA has been detected in the endocardium, but not the myocardium, of zebrafish embryos, suggesting that these proteins operate in an endothelial cell-autonomous signaling pathway.

Signaling through the HEG1 receptor and the CCM proteins of the CCM complex is required to regulate endothelial cell-cell association during formation of the cardiovascular system and for its integrity thereafter. Accordingly, loss of function of at least one of Heg1, CCM1, CCM2 and CCM3 leads to a malfunction of the CCM complex and to malformation of the components of the cardiovascular system, such as failure of nascent endothelial cells, defects in the heart, the aorta, lymphatic vessels, abnormal endothelial cell junctions, dilated heart phenotype early in development, heart failure associated with enlarged cardiac chambers in which the endocardium is covered by a thin layer of myocardial cells. In the context of the present invention, a protein required for a functional CCM complex includes Heg1, CCM1, CCM2 and CCM3 and any other protein known by the skilled person or that will be identified as being required for a functional CCM1 complex.

Arteriovenous malformation (AVM) is an abnormal connection between arteries and veins, bypassing capillary system. Arteriovenous malformation can appear in any location. In a preferred embodiment, it occurs in the central nervous system.

Hereditary hemorrhagic telangiectasia (HHT) is a rare autosomal dominant genetic disorder that leads to abnormal blood vessel formation in the skin, mucous membranes, and in organs such as lungs, liver, and brain.

As used herein, the term vascular lesion comprises cerebrovascular disease, which includes a variety of medical conditions that affect the blood vessels of the brain and the cerebral circulation and in particular stroke. Arteries supplying oxygen and nutrients to the brain are often damaged or deformed in these disorders. The most common presentation of cerebrovascular disease is an ischemic stroke or mini-stroke and sometimes a hemorrhagic stroke. Hypertension (high blood pressure) is the most important contributing risk factor for stroke and cerebrovascular diseases as it can change the structure of blood vessels and result in atherosclerosis. Atherosclerosis narrows blood vessels in the brain, resulting in decreased cerebral perfusion. Other risk factors that contribute to stroke include smoking and diabetes. Narrowed cerebral arteries can lead to ischemic stroke, but continually elevated blood pressure can also cause tearing of vessels, leading to a hemorrhagic stroke. A stroke usually presents with an abrupt onset of a neurologic deficit - such as hemiplegia (one-sided weakness), numbness, aphasia (language impairment), or ataxia (loss of coordination) - attributable to a focal vascular lesion. The neurologic symptoms manifest within seconds because neurons need a continual supply of nutrients, including glucose and oxygen, that are provided by the blood. Therefore, if blood supply to the brain is impeded, injury and energy failure is rapid. Besides hypertension, there are also many less common causes of cerebrovascular disease, including those that are congenital or idiopathic and include CADASIL (cerebral autosomal dominant arteriopathy with subcortical infarcts and leukoencephalopathy), aneurysms, amyloid angiopathy, arteriovenous malformations, fistulas, and arterial dissections. Many of these diseases can be asymptomatic until an acute event, such as a stroke, occurs. Cerebrovascular diseases can also present less commonly with headache or seizures. Any of these diseases can result in vascular dementia due to ischemic damage to the brain.

A stroke is a medical condition in which poor blood flow to the brain results in cell death. There are two main types of stroke: ischemic, due to lack of blood flow, and hemorrhagic, due to bleeding. Both result in parts of the brain not functioning properly. Signs and symptoms of a stroke may include an inability to move or feel on one side of the body, problems understanding or speaking, dizziness, or loss of vision to one side. Signs and symptoms often appear soon after the stroke has occurred. If symptoms last less than one or two hours it is known as a transient ischemic attack (TIA) or mini-stroke. A hemorrhagic stroke may also be associated with a severe headache. The symptoms of a stroke can be permanent. Long-term complications may include pneumonia or loss of bladder control. The main risk factor for stroke is high blood pressure. Other risk factors include tobacco smoking, obesity, high blood cholesterol, diabetes mellitus, a previous TIA, and atrial fibrillation. An ischemic stroke is typically caused by blockage of a blood vessel, though there are also less common causes. A hemorrhagic stroke is caused by either bleeding directly into the brain or into the space between the brain's membranes. Bleeding may occur due to a ruptured brain aneurysm. Diagnosis is typically based on a physical exam and supported by medical imaging such as a CT scan or MRI scan. A CT scan can rule out bleeding, but may not necessarily rule out ischemia, which early on typically does not show up on a CT scan. Other tests such as an electrocardiogram (ECG) and blood tests are done to determine risk factors and rule out other possible causes. Low blood sugar may cause similar symptoms.

The most common blood vessel abnormality/vascular malformation is arteriovenous malformation. Arteriovenous malformation is an abnormal connection between arteries and veins, bypassing the capillary system. This vascular anomaly is widely known because of its occurrence in the central nervous system (usually cerebral AVM), but can appear in any location. Although many AVMs are asymptomatic, they can cause intense pain or bleeding or lead to other serious medical problems. AVMs are usually congenital and belong to the RASopathies.

The term "cerebellar blood vessels" relates to blood vessels of the cerebellum.

In the context of the invention, the term "lowly perfused blood vessel" refers to blood vessels, with a low blood flow, meaning that the rate of blood flowing through these vessels is low, in particular compared to strongly perfused main blood vessels, such as the aorta. In lowly perfused blood vessels, hemodynamic forces are less pronounced and can lead to endothelial-to-mesenchymal transition (endMT) within lowly-perfuse blood vessels, which is a process common to many vascular pathologies.

Vascular lesions can be identified by visualizing the hollow or lumenized spaces of the cardiovascular system, which are the blood-filled spaces of the cardiovascular system. These can be visualized by various techniques known to a skilled person, for example by microangiography.

As used herein, acute vascular lesions refer to any acute symptoms associated with vascular lesions, such as bleeding, swelling wherein the inhibition of CBX7 terminates, halts, or decreases these symptoms. In a preferred embodiment, the inhibition of CBX7 leads to regression of vascular lesions.

As used herein, chronic vascular lesions refer to lesions that will develop sporadically over time wherein the patient may be asymptomatic. The inhibition of CBX7 has a preventive effect against chronic outbreaks, preferably, preventing the formation of vascular lesion.

Regression of vascular lesions shall mean decease or disappear of vascular lesions at some locations in including hemangiomas, vascular malformation, pyogenic granulomas.

Progression of vascular lesion shall mean increase of the vascular lesion at some locations or increase of the amounts of locations where vascular lesions occur. Prevention of the progression of vascular lesions refers to prevent the growth or increase of vascular lesions or stabilize the symptoms associated with vascular lesions.

Preventing the formation of vascular lesion shall mean reducing the probability of the formation of new vascular lesion at the location where vascular lesion occurs or the formation of vascular lesion at the location where no vascular lesion has occurred. In some embodiments, preventing formation of vascular lesion also refers to stop the formation of vascular lesion at the location where vascular lesion occurs or the formation of vascular lesion at the location where no vascular lesion has occurred. In another embodiments, preventing formation of vascular lesion refers to prevention reoccurrence of the vascular lesions.

As used herein, the terms "prevent," "preventing," "prevention," "prophylactic treatment," and the like, refer to reducing the probability of developing a disease or condition in a subject, who does not have, but is at risk of or susceptible to developing a disease or condition.

The terms "treat," "treated," "treating," "treatment," and the like are meant to refer to reducing or ameliorating a disorder and/or symptoms associated therewith. "Treating" may refer to administration of the combination therapy to a subject after the onset, or suspected onset, of a blood disorder. "Treating" includes the concepts of "alleviating", which refers to lessening the frequency of occurrence or recurrence, or the severity, of any symptoms or other ill effects related to a cancer and/or the side effects associated with cancer therapy. The term "treating" also encompasses the concept of "managing" which refers to reducing the severity of a particular disease or disorder in a patient or delaying its recurrence, e.g., lengthening the period of remission in a patient who had suffered from the disease. It is appreciated that, although not precluded, treating a disorder or condition does not require that the disorder, condition, or symptoms associated therewith be completely eliminated.

The term "subject" refers to an animal which is the object of treatment, observation, or experiment. By way of example only, a subject includes, but is not limited to, a mammal, including, but not limited to, a human or a non-human mammal, such as a non-human primate, bovine, equine, canine, ovine, feline, or fish, such as a zebrafish.

In the context of the invention, the term "pharmacological effect" relates to a biochemical or physiological effect of a drug or compound on a cell, tissue or organ of a subject. Such a pharmacological effect can be measured by determining the differences that occur in presence of the drug or compound as compared to its absence in a certain setup, such as an experimental setup.

The term "therapeutic effect" refers to some extent of relief of one or more of the symptoms of a disorder (e.g., a neoplasia or tumor) or its associated pathology. "Therapeutically effective amount" as used herein refers to an amount of an agent which is effective, upon single or multiple dose administration to the cell or subject, in prolonging the survivability of the patient with such a disorder, reducing one or more signs or symptoms of the disorder, preventing, or delaying, and the like beyond that expected in the absence of such treatment. "Therapeutically effective amount" is intended to qualify the amount required to achieve a therapeutic effect. A physician or veterinarian having ordinary skill in the art can readily determine and prescribe the "therapeutically effective amount" (e.g., ED50) of the pharmaceutical composition required. For example, the physician or veterinarian could start doses of the compounds of the invention employed in a pharmaceutical composition at levels lower than that required in order to achieve the desired therapeutic effect and gradually increase the dosage until the desired effect is achieved.

In one embodiment, the CBX7 inhibitor as used herein is in combination with a pharmaceutically acceptable carrier. Pharmaceutically acceptable carrier refers to a carrier or excipient or diluent that can be administered to a subject, together with an agent, and which does not destroy the pharmacological activity thereof and is non-toxic when administered in does sufficient to deliver a therapeutic amount of the agent.

Administration of the CBX7 inhibitor can be achieved by means of routes of administration known in the art. The route of administration of the inhibitor includes oral, subcutaneous, intravenous, intramuscular local administration. In some embodiments, the CBX7 inhibitor or in combination of pharmaceutically acceptable carrier is applied directly on the blood vessel, preferably for patient with CCM.

Simultaneous administration of one or more active agents shall mean administration of one or more agents at the same time. For example, in one embodiment, inhibition of CBX7 can be achieved by two or more CBX7 inhibitor at the same time. In other embodiment, CBX7 inhibitor can be administrated together other active agent useful for blood vessel disorder.

Sequential administration of one or more active agents shall mean administration of the therapeutic agents in a sequential manner. In one embodiment, each therapeutic agent is administered at a different time. In other embodiments, by administration of two or more therapeutic agents wherein at least two of the therapeutic agents are administered in a sequential manner which is a substantially simultaneous manner. Substantially simultaneous administration can be accomplished, for example, by administering to the subject a single dose having a fixed ratio of each therapeutic agent or in multiple, single doses for each of the therapeutic agents.

As used herein, the term "simultaneously" refers to administration of one or more agents at the same time. For example, in certain embodiments, different CBX7 inhibitors or CBX7 inhibitors in combination with other active agents useful for treating blood vessel disorder are administered at simultaneously. Simultaneously includes administration contemporaneously, that is during the same period of time. In certain embodiments, the one or more agents are administered simultaneously in the same hour, or simultaneously in the same day. Sequential or substantially simultaneous administration of each therapeutic agent can be affected by any appropriate route including, but not limited to, oral routes, intravenous routes, subcutaneous routes, intramuscular routes, direct absorption through mucous membrane tissues (e.g., nasal, mouth, vaginal, and rectal), and ocular routes (e.g., intravitreal, intraocular, etc.). The therapeutic agents can be administered by the same route or by different routes. For example, one component of a particular combination may be administered by intravenous injection while the other component(s) of the combination may be administered orally. The components may be administered in any therapeutically effective sequence.

In one embodiment, cerebral cavernous malformations are characterized by the presence of vascular lesions that can occur sporadically or through a familial condition from mutations in CCM1/KRIT1, CCM2/Malcavernin or CCM3/PDCD10. In some embodiment, a zebrafish *ccm2^{m201}* mutants wherein the phenotype of cardiovascular defect manifest, such as cardiac ballooning, are used for study of cerebral cavernous malformations. In other embodiments, *kri-1; ccm-3* double mutants in *C*. *elegans* are used for study of cerebral cavernous malformations.

In the context of the present disclosure, a zebrafish CCM2 mutant is *ccm2^{m201}* mutant disrupting the interaction between CCM1 and CCM2. The cardiac phenotype of *ccm2^{m201}* mutant is characterized by cardiac ballooning, endocardial over-proliferation, and by a lack of cardiac cushions that normally form by 48 postfertilization, as evidenced by the failure of endocardial cells to acquire a cuboidal shape and to express activated leukocyte cell adhesion molecule. Within the *ccm2^{m201}* mutant vasculature, the lateral dorsal aortas (LDAs) exhibited significant increases in EC numbers at 48 hrpf, providing evidence for increased angiogenesis signaling. Similarly, within the trunk region region of *ccm2^{m201}* mutants, the subintestinal vein (SIV) exhibited a higher density of vessel branch points, which is another established marker for increased angiogenesis (Renz et al., 2015).

Unless defined otherwise, all technical and scientific terms used herein have the same meaning as commonly understood by one of ordinary skill in the art to which this invention belongs. The practice of the present invention will employ, unless otherwise indicated, conventional techniques of chemistry, molecular biology, microbiology, recombinant DNA, and immunology, which are within the capabilities of a person of ordinary skill in the art. Such techniques are explained in the literature. See, e.g., J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements) Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons J. M. Polak and James O'D. McGee, 1990, In Situ Hybridization: Principles and Practice; Oxford University Press; M. J. Gait (Editor), 1984, Oligonucleotide Synthesis: A Practical Approach , Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies: A Laboratory Manual: Portable Protocol NO. I by Edward Harlow, David Lane, Ed Harlow (1999, Cold Spring Harbor Laboratory Press, ISBN 0-87969-544-7); Antibodies: A Laboratory Manual by Ed Harlow (Editor), David Lane (Editor) (1988, Cold Spring Harbor Laboratory Press, ISBN 0-87969-3,4-2), 1855. Handbook of Drug Screening, edited by Ramakrishna Seethala, Prabhavathi B. Fernandes (2001, New York, N.Y., Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3. Also, all publications, patent applications, patents and other references mentioned herein are incorporated by reference in their entirety.

### FIGURES

The following figures are presented to describe particular embodiments of the invention, without being limiting in scope.

### Brief description of the Figures:

**Figure 1****:** Various alleles of the zebrafish cbx7a locus
**Figure 2****:** Expression of the ccm2 mutant phenotype is conditional to the activity of Cbx7a.
**Figure 3****:** Mechanism of action of the two CBX7 inhibitors MS37452 and MS351
**Figure 4****:** Pharmacological treatment of ccm2 mutants of zebrafish with the CBX7 inhibitors MS37452 and MS351.

### Detailed description of the Figures:

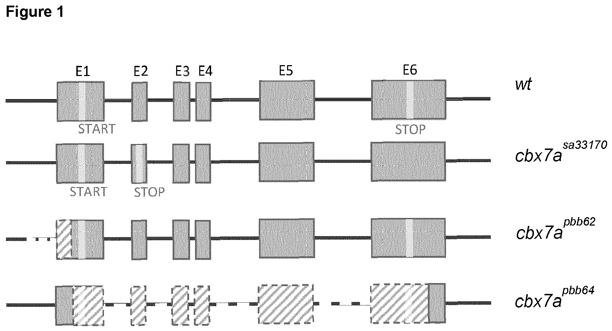

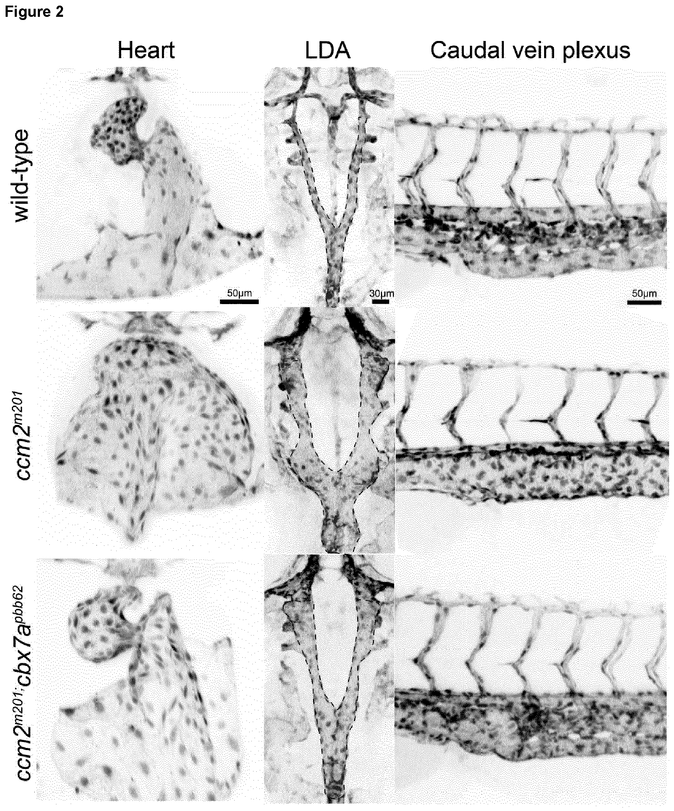

**Figure 1****:** To elucidate the role of Cbx7a in the zebrafish CCM disease model, we generated genetic strains of zebrafish carrying the *ccm2* mutation and the different mutant alleles of *cbx7a.* In *ccm2;cbx7a* double mutants the *ccm2* mutant phenotype was suppressed (Fig. 2). This phenotype is characterized by a ballooning and enlarged heart, an extended lateral dorsal aorta (LDA) and a caudal vein plexus that is lacking fenestration (as seen in wild-type). This finding revealed that Cbx7 has an important role in the expression of the CCM mutant phenotype.

**Figure 2****:** Shown is the effect of the cbx7apbb62 mutation (promoter deletion allele; a null allele of cbx7a) on the ccm2 mutant phenotype expression. The left column depicts the heart phenotypes of normal and ccm2 mutant animals. In comparison to wild type, the heart is enlarged and ballooning. In ccm2;cbx7a double mutants, the heart phenotype is strongly suppressed and reminiscent of wild-type. Similarly, the enlarged lateral dorsal aorta (LDA) of ccm2 mutants is strongly enlarged compared with wild type (marked with yellow). However, this phenotype is suppressed in ccm2;cbx7a double mutants. The posterior caudal vein plexus is shown in the right column. In wild-type, this vessel bed is highly fenestrated while a fenestration is completely lacking in the ccm2 mutant. The morphology of the caudal vein plexus is rescued and exhibits a fenestration in ccm2;cbx7a double mutants.

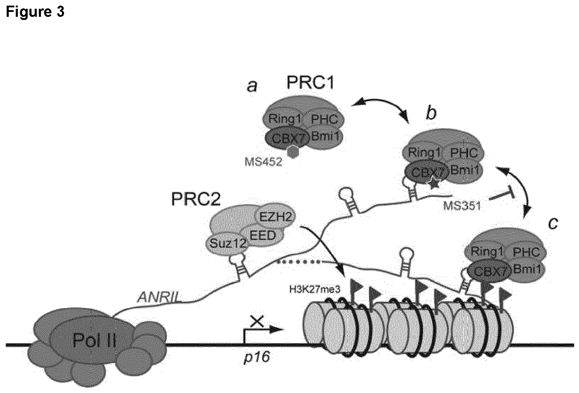

**Figure 3****:** The mechanism of action of the two CBX7 inhibitors MS37452 and MS351 according to Ren et al. (Structure-Guided Discovery of Selective Antagonists for the Chromodomain of Polycomb Repressive Protein CBX7. ACS Med Chem Lett. 2016, 7(6):601-5) is shown.

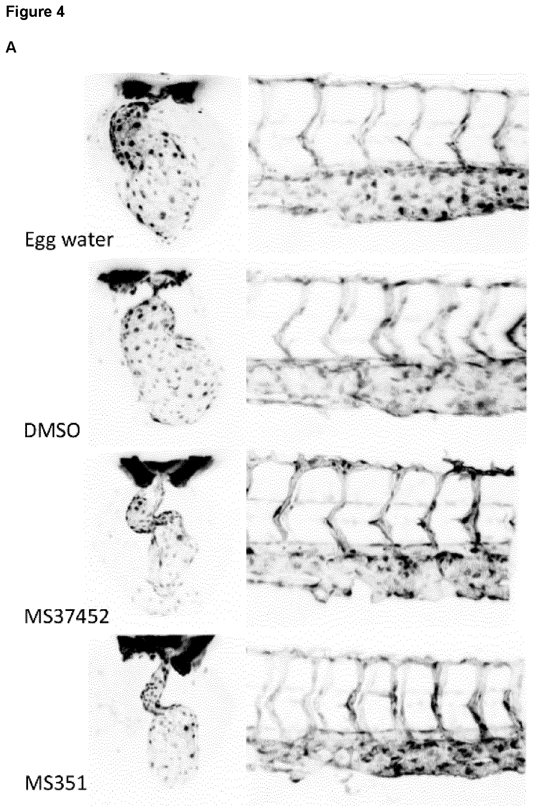

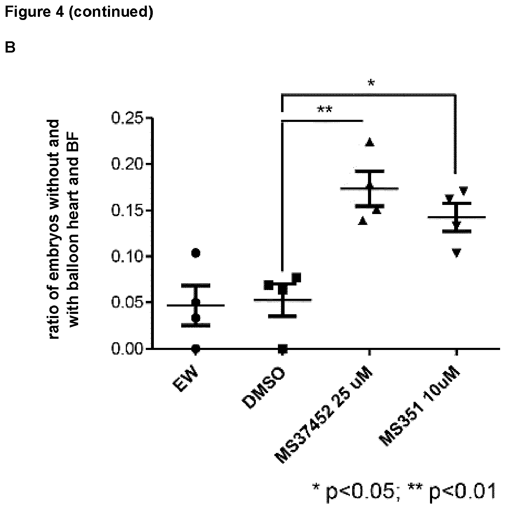

**Figure 4****:** A) the left column depicts heart phenotypes while the right column shows the region of the posterior caudal vein plexus. In *ccm2* mutants the heart is strongly enlarged and ballooning. In *ccm2* mutants treated with MS37452 or MS351, this phenotype is suppressed and is reminiscent of wild type. Similarly, the posterior caudal vein plexus in *ccm2* mutants is fenestrated after treatment with the inhibitors. Quantifications of these phenotype classes are in B).

### EXAMPLES

The following examples describe the treatment cerebral cavernous malformation (CCM) using inhibitors against CBX7 without being limiting in scope.

### Example 1

A well-established disease model of CCM in zebrafish for whole-transcriptome analyses was used and it has been detected that the gene cbx7a is highly expressed (strongly upregulated). In comparison the gene expression is almost not detectable in healthy animals. Based on this finding, we used CRISPR/Cas9-mediated mutagenesis to generate several knock-out mutations at this locus (deletion of the promoter region/ deletion of the entire coding region of the cbx7a locus). In addition, a point mutation at the cbx7a locus already existed (Fig. 1).

### Example 2

The phenotype expression of the *cbx7a^{pbb62}* mutation (promoter deletion allele; a null allele of *cbx7a*) on the *ccm2* mutant is shown in figure 2. The heart phenotypes of normal and ccm2 mutant animals is depicted in left column. In comparison to wild type, the heart is enlarged and ballooning. In *ccm2;cbx7a* double mutants, the heart phenotype is strongly suppressed and reminiscent of wild-type. Similarly, the enlarged lateral dorsal aorta (LDA) of *ccm2* mutants is strongly enlarged compared with wild type (marked with yellow). However, this phenotype is suppressed in *ccm2;cbx7a* double mutants. The posterior caudal vein plexus is shown in the right column. In wild type, this vessel bed is highly fenestrated while a fenestration is completely lacking in the *ccm2* mutant. The morphology of the caudal vein plexus is rescued and exhibits a fenestration in *ccm2;cbx7a* double mutants.

### Example 3

To elucidate whether a pharmacological suppression of Cbx7a has the same effect on the zebrafish CCM model, specific small compound inhibitors of CBX7 were tested (MS37452 and MS351). Both inhibitors block the binding of CBX7 to histone modifications on genomic DNA and suppress the epigenetic function of this protein within the Polycomb repressive complex (PRC1) (Fig. 3).

Both inhibitors suppress the expression of the CCM phenotype in zebrafish disease models (Fig. 4). The effects of both inhibitors are reminiscent of the genetic knockout of cbx7a in *ccm2* mutants.

Heart phenotypes is shown in Fig. 4A left column while the region of the posterior caudal vein plexus in Fig. 4A right column. In *ccm2* mutants the heart is strongly enlarged and ballooning. In *ccm2* mutants treated with MS37452 or MS351, this phenotype is suppressed and is reminiscent of wild type. Similarly, the posterior caudal vein plexus in *ccm2* mutants is fenestrated after treatment with the inhibitors. Quantifications of these phenotype classes are shown Fig. 4B. The y-axis represents the ratio of the amounts of embryos with suppressed ballooning heart and abnormal caudal vein plexus phenotype to the amounts of embryos with ballooning heart and abnormal caudal vein plexus phenotype.

### Example 4

Testing of MS37452 and MS351 in CCM models in mouse or in human *CCM*-deficient endothelial cell culture systems is performed verifying that CCM associated phenotypes are suppressed by CBX7 inhibition.

### Example 5

Further specific inhibitors of CBX7 are tested for their efficacy in various CCM models, in particular in zebrafish, as well as in mouse and human CCM-deficient endothelial cell culture systems.

### Reference

Abdelilah-Seyfried S, Tournier-Lasserve E, Derry WB. Blocking Signalopathic Events to Treat Cerebral Cavernous Malformations. Trends Mol Med. 2020 Sep;26(9):874-887. doi: 10.1016/j.molmed.2020.03.003. Epub 2020 Apr 3. PMID: 32692314.
Akers AL, Johnson E, Steinberg GK, Zabramski JM, Marchuk DA. Biallelic somatic and germline mutations in cerebral cavernous malformations (CCMs): evidence for a two-hit mechanism of CCM pathogenesis. Hum Mol Genet. 2009 Mar 1;18(5):919-30. doi: 10.1093/hmg/ddn430. Epub 2008 Dec 16. PMID: 19088123; PMCID: PMC2640209.
Baeyens N, Bandyopadhyay C, Coon BG, Yun S, Schwartz MA. Endothelial fluid shear stress sensing in vascular health and disease. J Clin Invest. 2016 Mar 1;126(3):821-8. doi: 10.1172/JCI83083. Epub 2016 Mar 1. PMID: 26928035; PMCID: PMC4767335.
Baeyens N, Schwartz MA. Biomechanics of vascular mechanosensation and remodeling. Mol Biol Cell. 2016 Jan 1;27(1):7-11. doi: 10.1091/mbc.E14-11-1522. PMID: 26715421; PMCID: PMC4694763.
Bao Z, Xu X, Liu Y, Chao H, Lin C, Li Z, You Y, Liu N, Ji J. CBX7 negatively regulates migration and invasion in glioma via Wnt/β-catenin pathway inactivation. Oncotarget. 2017 Jun 13;8(24):3904839063. doi: 10.18632/oncotarget.16587. PMID: 28388562; PMCID: PMC5503594.
Castro M, Lavina B, Ando K, Älvarez-Aznar A, Abu Taha A, Brakebusch C, Dejana E, Betsholtz C, Gaengel K. CDC42 Deletion Elicits Cerebral Vascular Malformations via Increased MEKK3-Dependent KLF4 Expression. Circ Res. 2019 Apr 12;124(8):1240-1252. doi: 10.1161/CIRCRESAHA.118.314300. PMID: 30732528.
Chapman EM, Lant B, Ohashi Y, Yu B, Schertzberg M, Go C, Dogra D, Koskimaki J, Girard R, Li Y, Fraser AG, Awad lA, Abdelilah-Seyfried S, Gingras AC, Derry WB. A conserved CCM complex promotes apoptosis non-autonomously by regulating zinc homeostasis. Nat Commun. 2019 Apr 17;10(1):1791. doi: 10.1038/s41467-019-09829-z. PMID: 30996251; PMCID: PMC6470173.
Craig HD, Günel M, Cepeda 0, Johnson EW, Ptacek L, Steinberg GK, Ogilvy CS, Berg MJ, Crawford SC, Scott RM, Steichen-Gersdorf E, Sabroe R, Kennedy CT, Mettler G, Beis MJ, Fryer A, Awad lA, Litton RP. Multilocus linkage identifiestwo new loci for a mendelian form of stroke, cerebral vernous malformation, at 7p15-13 and 3q25.2-27. Hum Mol Genet. 1998 Nov;7(12):1851-8. doi: 10.1093/hmg/7.12.1851. PMID: 9811928.
Cuttano R, Rudini N, Bravi L, Corada M, Giampietro C, Papa E, Morini MF, Maddaluno L, Baeyens N, Adams RH, Jain MK, Owens GK, Schwartz M, Lampugnani MG, Dejana E. KLF4 is a key determinant in the development and progression of cerebral cavernous malformations. EMBO Mol Med. 2016 Jan 1;8(1):6-24. doi: 10.15252/emmm.201505433. PMID: 26612856; PMCID: PMC4718159.
Dubovsky J, Zabramski JM, Kurth J, Spetzler RF, Rich SS, Orr HT, Weber JL. A gene responsible for cavernous malformations of the brain maps to chromosome 7q. Hum Mol Genet. 1995 Mar;4(3):453-8. doi: 10.1093/hmg/4.3.453. PMID: 7795602. Federico A, Sepe R, Cozzolino F, Piccolo C, lannone C, lacobucci 1, Pucci P, Monti M, Fusco A. The complex CBX7-PRMT1 has a critical rote in regulating E-cadherin gene expression and cell migration. Biochim Biophys Acta Gene Regul Mech. 2019 Apr;1862(4):509-521. doi: 10.1016/j.bbagrm.2019.02.006. Epub 2019 Feb 28. PMID: 30826432.
Fisher OS, Deng H, Liu D, Zhang Y, Wei R, Deng Y, Zhang F, Louvi A, Turk BE, Boggon TJ, Su B. Structure and vascular function of MEKK3-cerebral cavernous malformations 2 complex. Nat Commun. 2015 Aug 3;6:7937. doi: 10.1038/ncomms8937. PMID: 26235885; PMCID: PMC4526114.
Forzati F, Federico A, Pallante P, Abbate A, Esposito F, Malapelle U, Sepe R, Palma G, Troncone G, Scarf6 M, Arra C, Fedele M, Fusco A. CBX7 is a tumor suppressor in mice and humans. J Clin Invest. 2012 Feb;122(2):612-23. doi: 10.1172/JCI58620. Epub 2012 Jan 3. Erratum in: J Clin Invest. 2013 Feb 1;123(2):934. PMID: 22214847; PMCID: PMC3266782.
Johannes Jung, Sonja C Buisman, Ellen Weersing, Albertina Dethmers-Ausema, Erik Zwart, Hein Schepers, Mike R Dekker, Seka S Lazare, Franziska Hammerl, Yulia Skokova, Susanne M Kooistra, Karin Klauke, Raymond A Poot, Leonid V Bystrykh, Gerald de Haan; CBX7 Induces Self-Renewal of Human Normal and Malignant Hematopoietic Stem and Progenitor Cells by Canonical and Non-canonical Interactions; Cell Rep 2019 Feb 12;26(7):1906-1918.e8. doi: 10.1016/j.celrep.2019.01.050.
Macek Jilkova Z, Lisowska J, Manet S, Verdier C, Deplano V, Geindreau C, Faurobert E, Albigas-Rizo C, Duperray A. CCM proteins control endothelial r31 integrin dependent response to shear stress. Biol Open. 2014 Nov 28;3(12):1228-35. doi: 10.1242/bio.201410132. PMID: 25432514; PMCID: PMC4265761.
Maddaluno L, Rudini N, Cuttano R, Bravi L, Giampietro C, Corada M, Ferrarini L, Orsenigo F, Papa E, Boulday G, Tournier-Lasserve E, Chapon F, Richichi C, Retta SF, Lampugnani MG, Dejana E. EndMT contributes to the onset and progression of cerebral cavernous malformations. Nature. 2013 Jun 27;498(7455):492-6. doi: 10.1038/nature12207. Epub 2013 Jun 9. PMID: 23748444.
Mleynek TM, Chan AC, Redd M, Gibson CC, Davis CT, Shi DS, Chen T, Carter KL, Ling J, Blanco R, Gerhardt H, Whitehead K, Li DY. Lack of CCM1 induces hypersprouting and impairs response to flow. Hum Mol Genet. 2014 Dec 1;23(23):6223-34. doi: 10.1093/hmg/ddu342. Epub 2014 Jul 2. PMID: 24990152; PMCID: PMC4222362.
Offen C, Knox J, Boulday G, Eymery M, Haniszewski M, Neuenschwander M, Radetzki S, Vogt 1, Hahn K, De Luca C, Cardoso C, Hamad S, Igual Gil C, Roy P, Albiges-Rizo C, Faurobert E, von Kries JP, Campillos M, Tournier-Lasserve E, Derry WB, Abdelilah-Seyfried S. Systematic pharmacological screens uncover novel pathways involved in cerebral cavernous malformations. EMBO Mol Med. 2018 Oct;10(10):e9155. doi: 10.15252/emmm.201809155. PMID: 30181117; PMCID:PMC 6180302.
Ren AA, Snellings DA, Su YS, Hong CC, Castro M, Tang AT, Detter MR, Hobson N, Girard R, Romanos S, Lightle R, Moore T, Shenkar R, Benavides C, Beaman MM, Müller-Fielitz H, Chen M, Mericko P, Yang J, Sung DC, Lawton MT, Ruppert JM, Schwaninger M, Korbelin J, Potente M, Awad lA, Marchuk DA, Kahn ML. PIK3CA and CCM mutations fuel cavernomas through a cancer-like mechanism; Nature. 2021 Jun;594(7862):271-276. doi: 10.1038/s41586-021-03562-8. Epub 2021 Apr28. PMID: 33910229
Ren C, Morohashi K, Plotnikov AN, Jakoncic J, Smith SG, Li J, Zeng L, Rodriguez Y, Stojanoff V, Walsh M, Zhou MM. Small-molecule modulators of methyl-lysine binding for the CBX7 chromodomain. Chem Biol. 2015 Feb 19;22(2):161-8. doi: 10.1016/j.chembio1.2014.11.021. Epub 2015 Feb 5. PMID: 25660273; PMCID: PMC4336573.
Ren C, Smith SG, Yap K, Li S, Li J, Mezei M, Rodriguez Y, Vincek A, Aguilo F, Walsh MJ, Zhou MM. Structure-Guided Discovery of Selective Antagonists for the Chromodomain of Polycomb Repressive Protein CBX7. ACS Med Chem Lett. 2016 Feb 29;7(6):601-5. doi: 10.1021/acsmedchemlett.6b00042. PMID: 27326334; PMCID: PMC4904266.
Renz M, Offen C, Faurobert E, Rudolph F, Zhu Y, Boulday G, Duchene J, Mickoleit M, Dietrich AC, Ramspacher C, Steed E, Manet-Dupa S, Benz A, Hassel D, Vermot J, Huisken J, Tournier-Lasserve E, Felbor U, Sure U, Albiges-Rizo C, Abdelilah-Seyfried S. Regulation of 131 integrin-Klf2-mediated angiogenesis by CCM proteins. Dev Cell. 2015 Jan 26;32(2):181-90. doi:10.1016/j.devce1.2014.12.016. PMID: 25625207.
Simon JA, Kingston RE. Mechanisms of polycomb gene silencing: knowns and unknowns. Nat Rev Mol Cell Biol. 2009 Oct;10(10):697-708. doi: 10.1038/nrm2763. Epub 2009 Sep 9. PMID: 19738629.
Spiegler S, Rath M, Paperlein C, Felbor U. Cerebral Cavernous Malformations: An Update an Prevalence, Molecular Genetic Analyses, and Genetic Counselling. Mol Syndromol. 2018 Feb;9(2):60-69. doi: 10.1159/000486292. Epub 2018 Jan 25. PMID: 29593473; PMCID: PMC5836221.
Su W, Han HH, Wang Y, Zhang B, Zhou B, Cheng Y, Rumandla A, Gurrapu S, Chakraborty G, Su J, Yang G, Liang X, Wang G, Rosen N, Scher HI, Ouerfelli 0, Giancotti FG. The Polycomb Repressor Complex 1 Drives Double-Negative Prostate Cancer Metastasis by Coordinating Sternness and Immune Suppression. Cancer Cell. 2019 Aug 12;36(2):139-155.e10. doi: 10.1016/j.cce11.2019.06.009. Epub 2019 Jul 18. PMID: 31327655; PMCID: PMC7210785.
Uhlik MT, Abell AN, Johnson NL, Sun W, Cuevas BD, Lobel-Rice KE, Horne EA, Dell'Acqua ML, Johnson GL. Rac-MEKK3-MKK3 scaffolding for p38 MAPK activation during hyperosmotic shock. Nat Cell Biol. 2003 Dec;5(12):1104-10. doi: 10.1038/ncb1071. Epub 2003 Nov 23. PMID: 14634666.
Zhao Y, Liu XL, Huang JH, Yin AJ, Zhang H. MicroRNA-18a suppresses ovarian carcinoma progression by targeting CBX7 and regulating ERK/MAPK signaling pathway and epithelial-to-mesenchymal transition. Eur Rev Med Pharmacol Sci. 2020 May;24(10):5292-5302. doi: 10.26355/eurrev_20200521311. PMID: 32495862.
Zhen CY, Tatavosian R, Huynh TN, Due HN, Das R, Kokotovic M, Grimm JB, Lavis LD, Lee J, Mejia FJ, Li Y, Yao T, Ren X. Live-cell single-molecule tracking reveals co-recognition of H3K27me3 and DNA targets polycomb Cbx7-PRC1 to chromatin. Elife. 2016 Oct 10;5:e17667. doi: 10.7554/eLife.17667. PMID: 27723458; PMCID: PMC5056789.
Zhou Z, Rawnsley DR, Goddard LM, Pan W, Cao XJ, Jakus Z, Zheng H, Yang J, Arthur JS, Whitehead KJ, Li D, Zhou B, Garcia BA, Zheng X, Kahn ML. The cerebral cavernous malformation pathway controls cardiac development via regulation of endocardial MEKK3 signaling and KLF expression. Dev Cell. 2015 Jan 26;32(2):168-80. doi: 10.1016/j.devce1.2014.12.009. PMID: 25625206; PMCID: PMC4589864.
Zhou Z, Rawnsley DR, Goddard LM, Pan W, Cao XJ, Jakus Z, Zheng H, Yang J, Arthur JS, Whitehead KJ, Li D, Zhou B, Garcia BA, Zheng X, Kahn ML. Dev Cell. 2015 Jan 26;32(2):168-80. doi: 10.101 6/j.devcel.2014.12.009. PMID: 25625206; The cerebral cavernous malformation pathway controls cardiac development via regulation of endocardial MEKK3 signaling and KLF expression. Dev Cell. 2015 Jan 26;32(2):168-80. doi: 10.1016/j.devcel.2014.12.009.
Zhou Z, Tang AT, Wong WY, Bamezai S, Goddard LM, Shenkar R, Zhou S, Yang J, Wright AC, Foley M, Arthur JS, Whitehead KJ, Awad lA, Li DY, Zheng X, Kahn ML. Cerebral cavernous malformations arise from endothelial gain of MEKK3-KLF2/4 signalling. Nature. 2016 Apr 7;532(7597):122-6. doi: 10.1038/nature17178. Epub 2016 Mar 30.

## Claims

1. A chromobox protein homolog 7 (CBX7) inhibitor for use in the treatment and/or prevention of a blood vessel disorder.

2. The CBX7 inhibitor for use according to claim 1, wherein the blood vessel disorder is a vascular malformation.

3. The CBX7 inhibitor for use according to the preceding claim, wherein the vascular malformation is cerebral cavernous malformation (CCM), capillary malformation (CM), lymphedema (LE), arteriovenous malformation (AVM) or hereditary hemorrhagic telangiectasia (HHT), preferably CCM.

4. The CBX7 inhibitor for use according to any one of the preceding claims, wherein the blood vessel disorder is associated with increased Cbx7 mRNA and/or protein expression in tissues of the cardiovascular system.

5. The CBX7 inhibitor for use according to any one of the preceding claims, wherein the Cbx7 inhibitor is for use in the treatment of acute or chronic vascular lesions.

6. The CBX7 inhibitor for use according to any one of the preceding claims, wherein administration of the CBX7 inhibitor leads to regression of vascular lesions.

7. The CBX7 inhibitor for use according to any one of the preceding claims, wherein administration of the CBX7 inhibitor prevents progression of vascular lesion.

8. The CBX7 inhibitor for use according to any one of the preceding claims, wherein the administration of the CBX7 inhibitor prevents the formation of vascular lesion.

9. The CBX7 inhibitor for use according to any one of the preceding claims, wherein the inhibitor blocks/prevents the binding of CBX7 to histone modifications on genomic DNA.

10. The CBX7 inhibitor for use according to any one of the preceding claims, wherein the inhibitor is selected from MS37452, MS351, adapted L3MMBTL1 inhibitor, UNC3866, UNC4976, nucleic acid molecules blocking or preventing Cbx7 expression, such as a CBX7-specific shRNA or a CBX7-specific gRNA used in conjunction with an RNA guided endonuclease or any combination thereof.

11. The CBX7 inhibitor for use according to any one of the preceding claims, in combination with a pharmaceutically acceptable carrier.

12. The CBX7 inhibitor for use according to any one of the preceding claims, wherein the treatment and/or prevention further comprises simultaneous or sequential administration of one or more other active agents useful for treating the blood vessel disorder.

## Patentansprüche

1. Ein Chromobox-Protein-Homolog 7 (CBX7)-Inhibitor zur Verwendung in der Behandlung und/oder Vorbeugung einer Blutgefäßerkrankung.

2. Der CBX7-Inhibitor zur Verwendung gemäß Anspruch 1, wobei die Blutgefäßstörung eine vaskuläre Missbildung ist.

3. Der CBX7-Inhibitor zur Verwendung gemäß dem vorhergehenden Anspruch, wobei die vaskuläre Fehlbildung eine zerebrale kavernöse Fehlbildung (CCM), eine kapillare Fehlbildung (CM), ein Lymphödem (LE), eine arteriovenöse Fehlbildung (AVM) oder eine hereditäre hämorrhagische Teleangiektasie (HHT) ist, vorzugsweise CCM.

4. Der CBX7-Inhibitor zur Verwendung gemäß einem der vorhergehenden Ansprüche, wobei die Blutgefäßstörung mit einer erhöhten Cbx7-mRNA- und/oder Proteinexpression in Geweben des kardiovaskulären Systems verbunden ist.

5. Der CBX7-Inhibitor zur Verwendung gemäß einem der vorhergehenden Ansprüche, wobei der Cbx7-Inhibitor zur Verwendung in der Behandlung von akuten oder chronischen Gefäßläsionen verwendet wird.

6. Der CBX7-Inhibitor zur Verwendung gemäß einem der vorhergehenden Ansprüche, wobei die Verabreichung des CBX7-Inhibitors zur Rückbildung von Gefäßläsionen führt.

7. Der CBX7-Inhibitor zur Verwendung gemäß einem der vorhergehenden Ansprüche, wobei die Verabreichung des CBX7-Inhibitors die Progression einer vaskulären Läsion verhindert.

8. Der CBX7-Inhibitor zur Verwendung gemäß einem der vorhergehenden Ansprüche, wobei die Verabreichung des CBX7-Inhibitors die Bildung einer vaskulären Läsion verhindert.

9. Der CBX7-Inhibitor zur Verwendung gemäß einem der vorhergehenden Ansprüche, wobei der Inhibitor die Bindung von CBX7 an Histonmodifikationen auf genomischer DNA blockiert/verhindert.

10. Der CBX7-Inhibitor zur Verwendung gemäß einem der vorhergehenden Ansprüche, wobei der Inhibitor ausgewählt ist aus MS37452, MS351, einem angepassten L3MMBTL1-Inhibitor, UNC3866, UNC4976, Nukleinsäuremolekülen, die die Cbx7-Expression blockieren oder verhindern, wie eine CBX7-spezifische shRNA oder eine CBX7-spezifische gRNA, die in Verbindung mit einer RNA-gesteuerten Endonuklease verwendet wird, oder einer Kombination davon.

11. Der CBX7-Inhibitor zur Verwendung gemäß einem der vorhergehenden Ansprüche in Kombination mit einem pharmazeutisch akzeptablen Träger.

12. Der CBX7-Inhibitor zur Verwendung gemäß einem der vorhergehenden Ansprüche, wobei die Behandlung und/oder Vorbeugung zusätzlich die gleichzeitige oder aufeinanderfolgende Verabreichung eines oder mehrerer anderer zur Behandlung der Blutgefäßerkrankung nützlicher Wirkstoffe umfasst.

## Revendications

1. Inhibiteur de l'homologue de la protéine chromobox 7 (CBX7) destiné à être utilisé dans le traitement et/ou la prévention d'une maladie des vaisseaux sanguins.

2. Inhibiteur de CBX7 destiné à être utilisé selon la revendication 1, dans lequel la maladie des vaisseaux sanguins est une malformation vasculaire.

3. Inhibiteur de CBX7 destiné à être utilisé selon la revendication précédente, dans lequel la malformation vasculaire est une malformation caverneuse cérébrale (CCM), une malformation capillaire (CM), un lymphœdème (LE), une malformation artérioveineuse (MAV) ou une télangiectasie hémorragique héréditaire (HHT), de préférence une CCM.

4. Inhibiteur de CBX7 destiné à être utilisé selon l'une quelconque des revendications précédentes, dans lequel la maladie des vaisseaux sanguins est associée à une expression accrue de l'ARNm et/ou de la protéine de CBX7 dans les tissus du système cardiovasculaire.

5. Inhibiteur de CBX7 destiné à être utilisé selon l'une quelconque des revendications précédentes, dans lequel l'inhibiteur de CBX7 est destiné à être utilisé dans le traitement de lésions vasculaires aiguës ou chroniques.

6. Inhibiteur de CBX7 destiné à être utilisé selon l'une quelconque des revendications précédentes, dans lequel l'administration de l'inhibiteur de CBX7 conduit à une régression des lésions vasculaires.

7. Inhibiteur de CBX7 destiné à être utilisé selon l'une quelconque des revendications précédentes, dans lequel l'administration de l'inhibiteur de CBX7 empêche la progression de la lésion vasculaire.

8. Inhibiteur de CBX7 destiné à être utilisé selon l'une quelconque des revendications précédentes, dans lequel l'administration de l'inhibiteur de CBX7 empêche la formation de la lésion vasculaire.

9. Inhibiteur de CBX7 destiné à être utilisé selon l'une quelconque des revendications précédentes, dans lequel l'inhibiteur bloque/empêche la liaison de CBX7 aux modifications d'histone sur l'ADN génomique.

10. Inhibiteur de CBX7 destiné à être utilisé selon l'une quelconque des revendications précédentes, dans lequel l'inhibiteur est choisi parmi MS37452, MS351, un inhibiteur de L3MMBTL1 adapté, UNC3866, UNC4976, des molécules d'acide nucléique bloquant ou empêchant l'expression de CBX7, telles qu'un shARN spécifique de CBX7 ou un ARNg spécifique de CBX7 utilisé conjointement avec une endonucléase guidée par ARN ou une quelconque combinaison de ceux-ci.

11. Inhibiteur de CBX7 destiné à être utilisé selon l'une quelconque des revendications précédentes, en combinaison avec un support pharmaceutiquement acceptable.

12. Inhibiteur de CBX7 destiné à être utilisé selon l'une quelconque des revendications précédentes, dans lequel le traitement et/ou la prévention comprend en outre l'administration simultanée ou séquentielle d'un ou plusieurs autres agents actifs utiles pour le traitement de la maladie des vaisseaux sanguins.
